# EUROPÄISCHE PATENTANMELDUNG

(11) **EP 2 617 456 A1**
(43) Veröffentlichungstag der Anmeldung: **24.07.2013**
(21) Anmeldenummer: 12192385.8
(22) Anmeldetag: 13.11.2012
(51) Int. Cl.: A61M 25/10, A61B 19/00, A61M 25/01, A61B 5/00, A61M 25/00

(54) **Katheter und Katheteranordnung**

(30) Priorität: 19.01.2012 US 201261588192 P
(71) Anmelder: VascoMed GmbH, 79589 Binzen (DE)
(72) Erfinder: Knorr, Stefan, 10119 Berlin (DE); Weiss, Ingo, 12435 Berlin (DE); Fandrey, Stephan, 8910 Affoltern am Albis (CH); Geistert, Wolfgang, 79618 Rheinfelden (DE)
(74) Vertreter: Lindner-Vogt, Karin L.

(57) **Zusammenfassung**

Katheter (10) mit einem langgestreckten Katheterkörper (13), der in Bezug auf eine Gebrauchslage ein distales (10d) und ein proximales Ende (10p) hat, wobei der Katheterkörper einen Innenschlauch (15) und einen diesem gegenüber verschiebbaren Außenschlauch (17) umfasst und am distalen Ende ein Deformationskörper (11) angeordnet ist, der in seinem distalen Bereich mit dem distalen Ende des Innenschlauches und in seinem proximalen Bereich mit dem distalen Ende des Außenschlauches verbunden ist.

## Beschreibung

Die Erfindung betrifft einen Katheter mit einem langgestreckten Katheterkörper, der in Bezug auf eine Gebrauchslage ein distales und ein proximales Ende hat. Sie betrifft des Weiteren eine Katheteranordnung mit einem solchen Katheter und einer daran angeschlossenen Messeinrichtung.

In der medizinischen Praxis ist eine Vielzahl verschieden konstruierter Katheter oder katheterähnlicher Einrichtungen (z.B. Elektrodenleitungen) bekannt und in massenhaftem Gebrauch. Sie werden teilweise von erfahrenen Spezialisten, teilweise aber auch von Ärzten oder auch medizinischem Personal ohne spezielle Kenntnisse und Erfahrung angewendet. Schädigungen oder Beeinträchtigungen des Patienten müssen gleichwohl zuverlässig ausgeschlossen werden.

Bekannte Katheter mit einer Kunststoff- oder Metallspitze weisen eine Perforationsgefahr auf. Um die Flächenpressung und damit die Perforationsgefahr gering zu halten, müssen bei der Steifheit des Katheterschaftes und der Katheterspitze Kompromisse eingegangen werden. Diese Kompromisse schränken u. U. die Manövrierfähigkeit und die Lagestabilität des Katheters ein.

Der Erfindung liegt daher die Aufgabe zugrunde, einen verbesserten Katheter bereitzustellen, der ohne die Gefahr von Schädigungen oder Beeinträchtigungen des Patienten auch von weniger Geübten eingesetzt werden kann und dessen Konstruktion, insbesondere hinsichtlich der Flexibilität, dennoch ohne weiteres den im klinischen Einsatz bestehenden Anforderungen gerecht wird.

Diese Aufgabe wird durch einen Katheter mit den Merkmalen des Anspruchs 1 gelöst. Mit der Erfindung wird des Weiteren eine Katheteranordnung mit den Merkmalen des Anspruchs 15 bereitgestellt.

Die Erfindung geht von dem Gedanken aus, bekannte Katheteraufbauten durch ein spezielles Perforationsschutzelement zu modifizieren. Sie schließt weiter den Gedanken ein, einen Deformationskörper am distalen Ende des Katheters vorzusehen. Schließlich gehört zur Erfindung der Gedanke eines (mindestens) zweischichtigen Aufbaus des Katheterkörpers, wobei der Katheter über einen äußeren und einen inneren Schlauch verfügt, die über einen Deformationskörper miteinander verbunden sind, wobei der innere Schlauch mit dem distalen Ende des Deformationskörper und der äußere Schlauch mit dem proximalen Ende des Deformationskörpers verbunden ist. Wenn eine axiale Kraft auf die Katheterspitze einwirkt, wird der Deformationskörper verformt und der innere Schlauch schiebt sich teleskopartig in den äußeren Schlauch.

Eine Ausführung, bei der der Deformationskörper einen größeren Durchmesser als die eigentliche Katheterspitze hat, hat den Vorteil, dass die Auflagefläche des Katheters am Gewebe und somit die Flächenpressung zusätzlich verringert wird.

In einer weiteren Ausführung ist der Deformationskörper als Ballon mit Fluidfüllung ausgebildet. Eine Ausgestaltung dieser Variante sieht vor, dass der Ballon über einen Fluidkanal im Katheterkörper in Fluidverbindung mit einem Fluidanschluss am proximalen Ende des Katheters steht. Noch spezieller kann diese Variante so ausgestaltet sein, dass der Ballon mit einem elastisch kompressiblen oder inkompressiblen Fluid gefüllt ist.

Ein Ballon als Deformationskörper bietet den Vorteil, dass er im deflatierten Zustand eingeführt werden kann und so kaum mehr Einführquerschnitt benötigt als der Katheterschaft. Außerdem kann bei Verwendung eines Ballons durch die beim Andrücken an das Gewebe erfolgende Druckänderung und Messung derselben gleichzeitig auf die Andruckfläche und/oder die Andruckkraft geschlossen werden.

In einer anderen Variante ist der Deformationskörper aus einem elastisch kompressiblen Material gebildet.

In jeder der vorgenannten Ausführungen, aber auch als selbständig funktionsfähige Ausführung, kann der Deformationskörper eine im Wesentlichen axial wirkende Druckfedereinrichtung, insbesondere eine Metall- oder Kunststofffeder, aufweisen.

Eine weitere Ausgestaltung sieht vor, dass der Deformationskörper eine formelastische Hülle oder Mantelschicht aufweist.

Im Hinblick auf die funktionswesentlichen mechanischen Eigenschaften gibt es also die folgenden Varianten: Der Deformationskörper
- kann ein Ballon sein, der mit einem Medium gefüllt wird, dessen Druck die Härte und somit die Federkraft des Ballons bestimmt, oder
- kann aus einem weichen Kunststoff oder einem Schaum gefertigt sein, welcher durch seine elastischen Eigenschaften die Federkraft bestimmt, oder
- kann auch eine gewöhnliche Feder sein, welche durch ihre Federkonstante die Federkraft bestimmt.

Im Einsatz des Katheters unterliegt der Deformationskörper beim Andrücken an die Wandung eines Gefäßes oder Hohlorgans einer Druckänderung.

Die Druckänderung kann distal oder proximal gewandelt werden. Mögliche Realisierungen der Druckmessung sind:
- Übertragung des Druckes im hydraulischen System vom distalen zum proximalen Ende des Katheters. Die Wandlung der Druckinformation erfolgt am proximalen Ende (z.B. im Ablationsgerät oder in der Pumpe).
- Der Druck wird im distalen Ende des Katheters konvertiert und elektrisch oder optisch zum proximalen Ende übertragen. Zur Druckmessung können diverse Methoden angewendet werden.
- Es können die optischen Eigenschaften des Mediums im Ballon gemessen werden, um so Rückschlüsse auf die Deformation des Deformationskörpers und so auf den Andruck des Katheters an das Gewebe zu ziehen.
- Wird als Deformationskörper ein weicher Kunststoff oder ein Schaum benutzt, kann die beim Andrücken an das Gewebe erfolgende Druckänderung zu einer Änderung der elektrischen Eigenschaften des Deformationskörpers (Widerstand, Kapazität,...) führen, welche ebenfalls gemessen und zur Ermittlung der Andruckfläche und/oder Andruckkraft genutzt werden können.

In einer weiteren Ausführung ist der Deformationskörper mehrteilig aus mehreren Kammern oder Teilkörpern ausgebildet, wobei die Kammern oder Teilkörper unterschiedliches Deformationsverhalten und/oder separate Messmittel oder -anschlüsse zur Erfassung einer spezifisch auf sie ausgeübten Andruckkraft aufweisen.

Hierbei ist insbesondere vorgesehen, dass die Kammern jeweils über einen Fluidkanal im Katheterkörper in Fluidverbindung mit einem Fluidanschluss am proximalen Ende des Katheters stehen oder die Teilkörper optisch oder elektrisch wirkende Messmittel oder -anschlüsse aufweisen.

In einer weiteren Ausführung ist der Katheter als Elektrodenleitung mit mindestens einer auf dem Deformationskörper oder am distalen Ende des Innenschlauches angeordneten Elektrode ausgebildet. Im zuletzt genannten Fall ist die Elektrode vom Deformationskörper umgeben. Hierbei ist insbesondere die oder mindestens eine Elektrode elastisch deformierbar, insbesondere aus leitfähigem Kunststoff gebildet.

Vorteile und Zweckmäßigkeiten der Erfindung ergeben sich im Übrigen aus der nachfolgenden skizzenartigen Beschreibung von Ausführungsbeispielen anhand der Figuren. Von diesen zeigen:
Fig. 1A bis 1D schematische Darstellungen (Seitenansichten bzw. Querschnittsdarstellungen) einer Ausführungsform des erfindungsgemäßen Katheters im Ausgangszustand bzw. deformierten Zustand des Katheterendes,
Fig. 2A und 2B schematische perspektivische Darstellungen einer weiteren Ausführungsform des Katheters,
Fig. 3A und 3B schematische perspektivische Darstellungen einer weiteren Ausführungsform des Katheters,
Fig. 4A und 4B eine perspektivische Darstellung bzw. Querschnittsdarstellung einer weiteren Ausführungsform des erfindungsgemäßen Katheters,
Fig. 5 eine schematische Darstellung einer Ausführungsform des vorgeschlagenen Katheters als bipolare Elektrodenleitung und
Fig. 6A und 6B Prinzipskizzen von Ausführungsformen der erfindungsgemäßen Katheteranordnung.

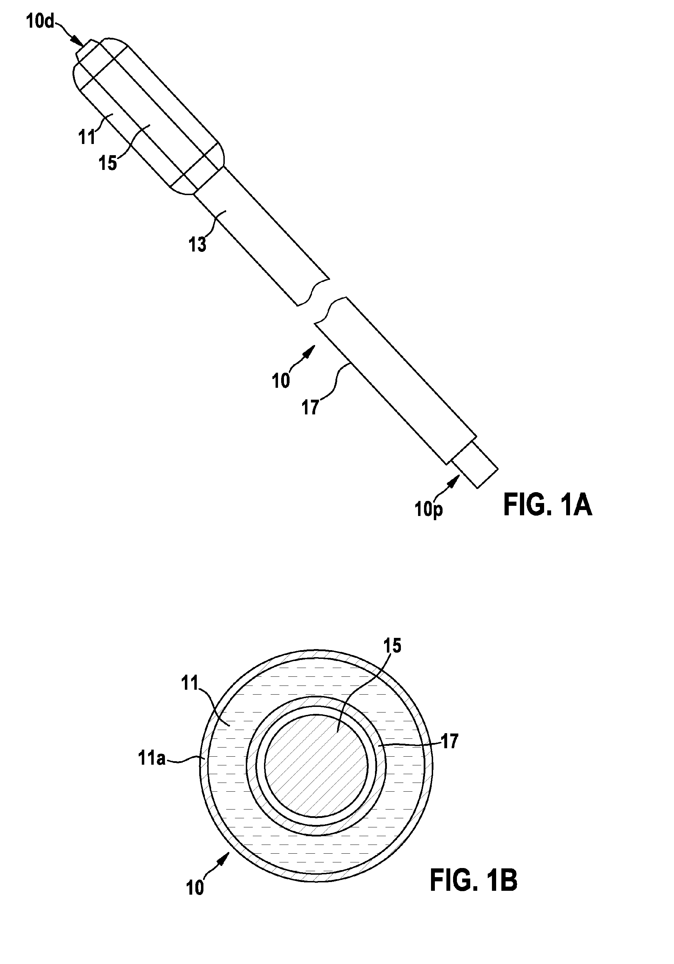

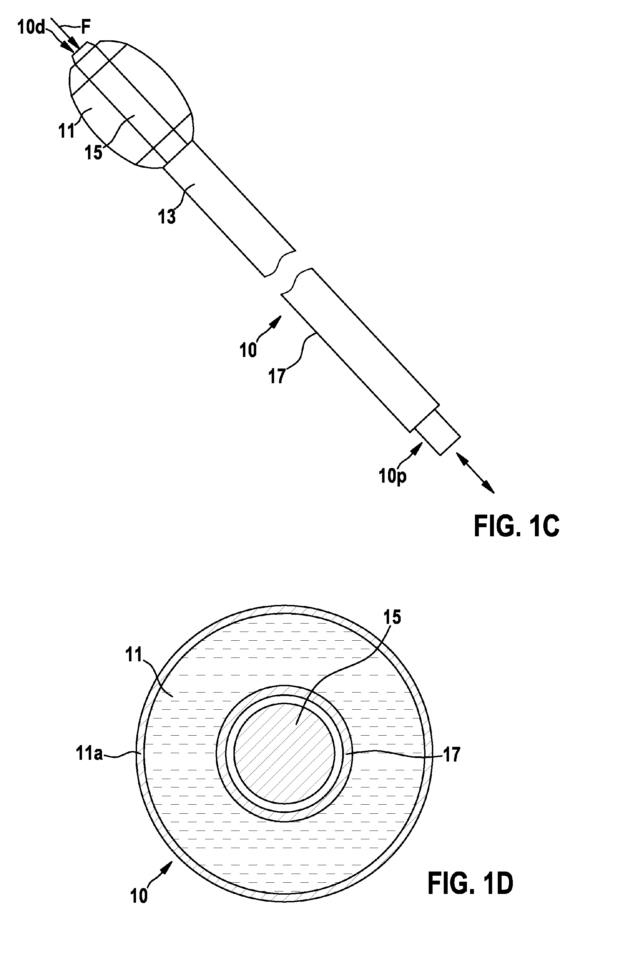

Fig. 1A bis 1D zeigen schematisch einen Katheter 10 mit einem distalen Ende 10d und einem proximalen Ende 10p, bei dem am distalen Ende ein Deformationskörper 11 angebracht ist. In der dargestellten Ausführung handelt es sich dabei um einen Ballon mit einer formelastischen Hülle 11a, die sich bei Wandkontakt des distalen Katheterendes 10d, wie Fig. 1C zeigt, bauchig verformt. Der Ballon 11 ist mit einem geeigneten Fluid gefüllt, wobei es sich um ein unter Druck stehendes Gas, grundsätzlich aber auch um eine Flüssigkeit oder eine Schüttung aus (insbesondere kompressiblen) Feststoffteilchen handeln kann.

Ein Katheterkörper 13 des Katheters 10 umfasst einen Innenschlauch 15 und einen diesem gegenüber proximal verschiebbaren Außenschlauch 17. Am distalen Ende des Innenschlauches ist das distale Ende des Ballons 11 befestigt, und am distalen Ende des Außenschlauches ist das proximale Ende des Ballons 11 befestigt. Aufgrund der Verschiebbarkeit von Innen- und Außenschlauch relativ zueinander führt, wie in Fig. 1C zu erkennen, die Einwirkung einer Druckkraft F auf das distale Ende des Katheters neben der Deformation des Ballons 11 zu einer Verschiebung des Innenschlauches 15 relativ zum Außenschlauch 17 in proximaler Richtung. Diese Verschiebung kann detektiert und ausgewertet werden; zum messtechnischen Einsatz des vorgeschlagenen Katheters siehe im Übrigen die Ausführungen weiter unten.

Wird als Inflationsmedium eine Flüssigkeit oder ein Gas (z.B. Kochsalzlösung oder Kohlendioxid) genutzt, so kann dieses über einen der Schläuche - aus derzeitiger Sicht bevorzugt über den Außenschlauch 17 - zugeführt und das Deformationsverhalten des distalen Katheterendes durch den eingestellten Druck eingestellt werden. Ist der Ballon dehnbar, kann über den Fluiddruck auch seine Größe wunschgemäß eingestellt werden.

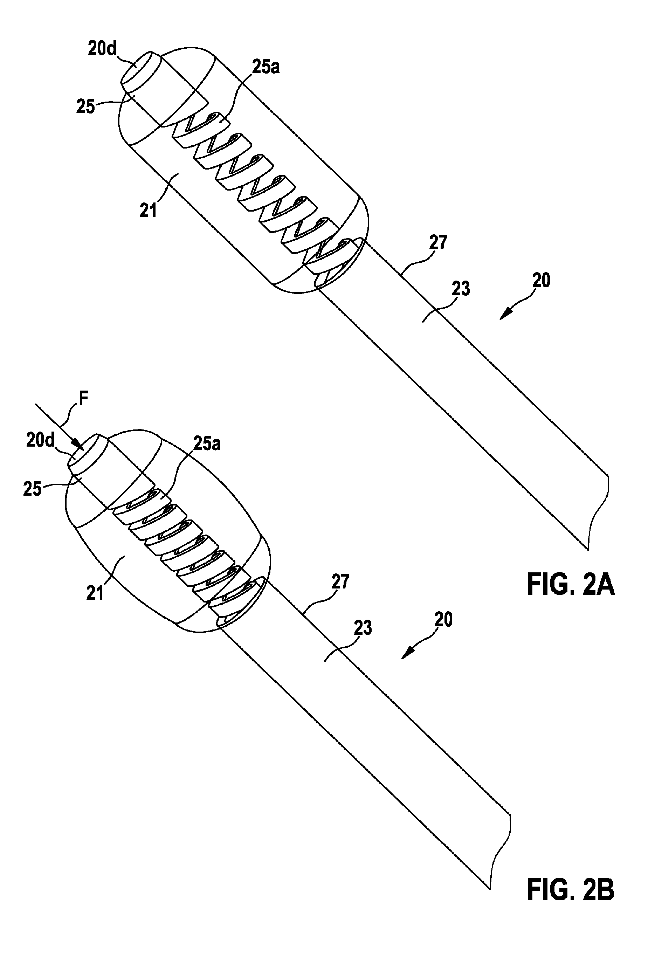

Fig. 2A und 2B zeigen einen weiteren erfindungsgemäßen Katheter, wobei an Fig. 1A bis 1D angelehnte Bezugsziffern gewählt sind und weiter oben im Bezug auf die erste Ausführung gegebene Erläuterungen hier nicht wiederholt werden. 2A zeigt einen Ausgangszustand und Fig. 2B einen Gebrauchszustand unter Einwirkung einer distal wirkenden Kraft F.

Bei dieser Ausführung ist ein Bereich des Innenschlauches 25 nahe dessen distalem Ende durch einen entsprechenden spiralig verlaufenden Ausschnitt in ein Druckfederelement 25a umfunktioniert worden, welches zusammen mit der Formelastizität der Hülle des Deformationskörpers 21 und/oder dessen kompressibler Füllung die distal wirkende Andruckkraft F mindestens bis zu einem gewissen Grade aufnimmt. Hierbei verschiebt sich das distale Katheterende nach proximal, ohne dass eine Verschiebbarkeit des gesamten Innenschlauches relativ zum Außenschlauch gegeben sein muss. Das Vorsehen eines Druckfederbereiches bzw. -elementes kann aber auch mit einer Verschiebbarkeit des gesamten Innenschlauches gegenüber dem Außenschlauch kombiniert sein. Statt einen Abschnitt des Innenschlauches als Druckfederelement auszugestalten, kann zwischen Innen- und Außenschlauch auch ein separates Druckfederelement (beispielsweise auch aus einem Metall) vorgesehen sein.

Wie bei der erstgenannten Ausführung wird durch die Verschiebung in Verbindung mit der Deformation des Deformationskörpers die Flächenpressung am distalen Katheterende und somit auch eine Perforationsgefahr vermindert.

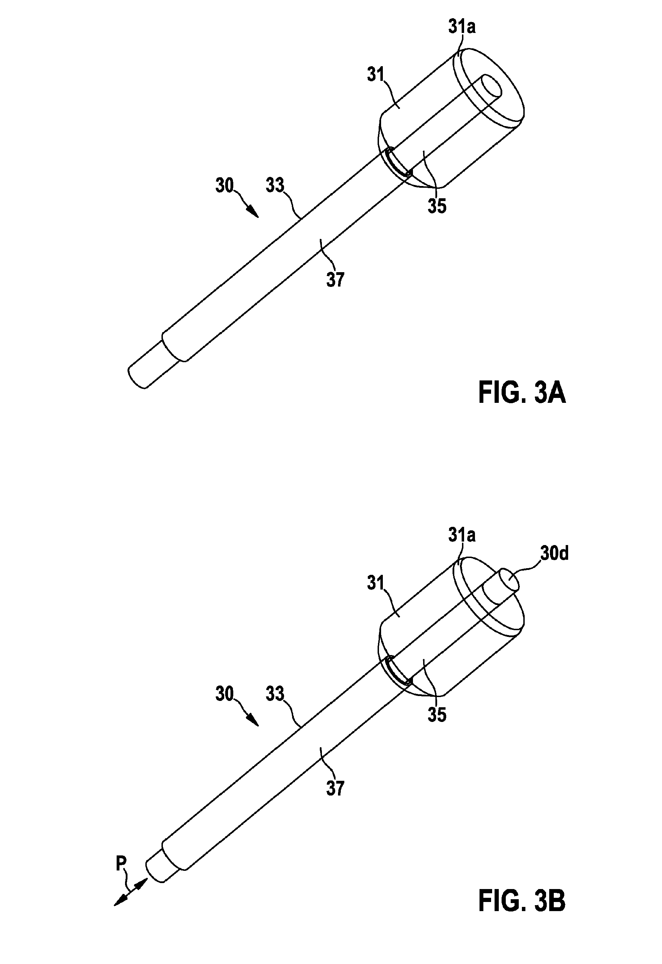

Fig. 3A und 3B zeigen eine etwas anders funktionierende Ausführung. Der Deformationskörper 31 hat hier Zylinderform und an der distalen Stirnfläche einen Kragen 31 a, mit dem sich das Katheter bei Wandkontakt an der Gefäßwand ausrichten kann. Durch axialen Druck p wird, wie in Fig. 3B skizziert, dann der Innenschlauch 35 über die hierdurch definierte Kontaktfläche geringfügig vorgeschoben, womit das distale Katheterende 30d etwas in das umgebende Körpergewebe eingedrückt wird. Über den Vorschub kann eine gewünschte Kontaktfläche und -kraft eingestellt werden. Ist am distalen Ende eine (nicht dargestellte) Elektrode angeordnet, so kann hierüber eine gewünschte Elektrode-Gewebekontakt eingestellt werden.

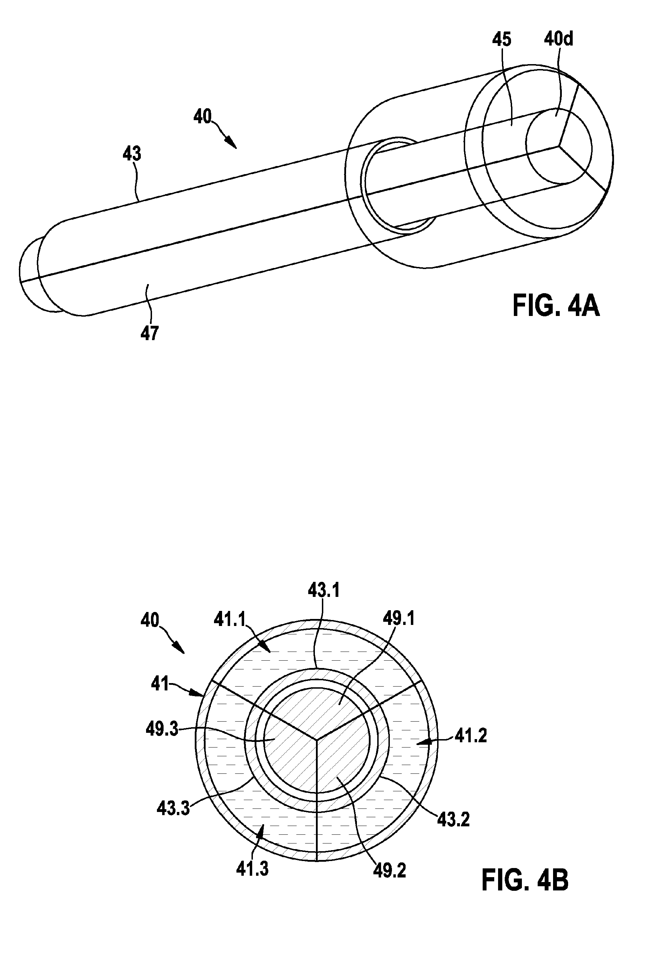

Fig. 4A und 4B zeigen als weitere Ausführung der Erfindung schematisch einen Drei-Kammer-Katheter 40, bei dem sowohl der Katheterkörper 43 als auch der Deformationskörper 41 in drei Kammern unterteilt ist. Dies ist am besten in Fig. 4B zu erkennen, wo die Kammern 41.1, 41.2 und 41.3 des Deformationskörpers auch ebenso einzeln bezeichnet sind wie die Teile 43.1, 43.2 und 43.3 des Katheterkörpers. Es ist hier auch zu erkennen, dass zu jeder Kammer ein eigener Fluidkanal 49.1, 49.2 bzw. 49.3 im Inneren des Innenkatheters gehört.

Bekommt das Katheter 40 im Gebrauch Wandkontakt an der Wandung eines Gefäßes oder Hohlorgans, so wirkt sich dieser Wandkontakt für die einzelnen Kammern des Deformationskörpers unterschiedlich aus, und an einem mit dem jeweiligen Fluidkanal verbundenen (nicht dargestellten) Fluidausgang lässt sich ein spezifischer Druckanstieg abgreifen. Daraus kann dann neben der Gesamtkraft auch die Richtung bestimmt und damit eine zusätzliche Kenntnis der Lage des Katheters gewonnen werden.

Ist in einer Abwandlung der Deformationskörper nicht als Drei-Kammern-Ballon, sondern etwa aus drei separaten Schaumstoffteilen gebildet, so ließe sich etwa über die Erfassung des elektrischen Widerstandes der einzelnen Teile eine ähnliche Bestimmung vornehmen.

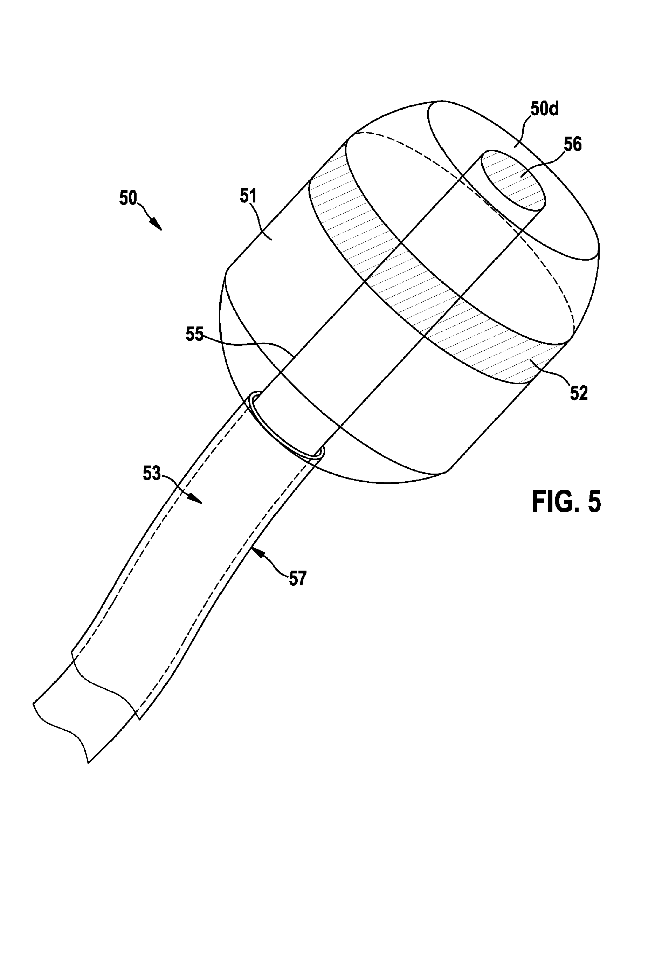

Fig. 5 zeigt schematisch das distale Ende eines erfindungsgemäßen Elektrodenkatheters 50 mit einem Deformationskörper 51 und Katheterkörper 53, der einen Innenschlauch 55 und Außenschlauch 57 umfasst und in seinem distalen Bereich zwei Elektroden trägt, die zur Gewebestimulation und/oder zum Abfühlen von Gewebepotentialen eingesetzt werden können. Am distalen Ende 50d des Elektrodenkatheters ist eine Spitzenelektrode 56 vorgesehen, die beispielsweise durch eine Metallbeschichtung des distalen Endes des Innenschlauches 55 erzeugt sein kann. Daneben ist auf dem Umfang des Deformationskörpers 51 eine Ringelektrode 52 vorgesehen, die etwa aus einem dehnbaren leitfähigen Kunststoff gebildet sein kann. Alternativ ist auch beispielsweise das Vorsehen eines mäanderförmigen Metallstreifens o. ä. möglich.

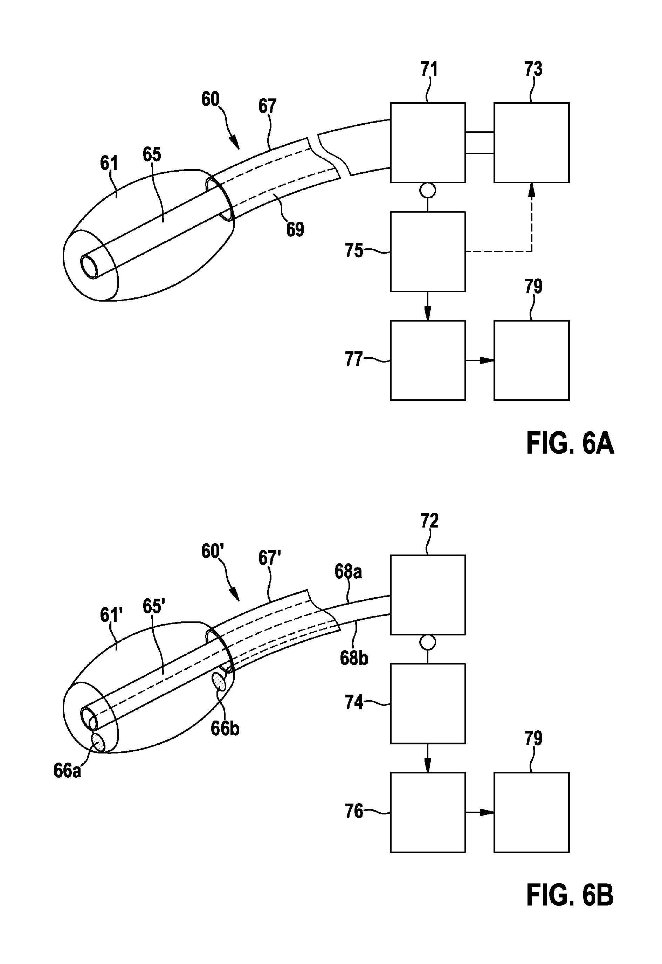

Fig. 6A zeigt eine Ausführung einer Katheteranordnung, die einen Katheter 60, welcher gemäß einer der weiter oben erläuterten Varianten ausgeführt sein kann, in Verbindung mit Peripheriegeräten umfasst. Zwischen dem Innenschlauch 65 und der Hülle des Außenschlauches 67 ist hier ein Fluidkanal 69 vorgesehen, über den das Innere des als fluidgefüllter Ballon ausgeführten Deformationskörpers 61 mit einem Fluidanschluss 71 am proximalen Ende des Katheters in Verbindung steht. Über den Fluidanschluss 71 liefert eine Fluidquelle 73 (etwa eine Kohlendioxidflasche mit regelbarem Ventil oder eine Gaspumpe) ein geeignetes Fluid zum Füllen des Ballons 61 mit vorbestimmtem Druck.

Des Weiteren wird am Fluidanschluss 71 mittels eines Drucksensors 75 der Innendruck im Ballon erfasst. Der erfasste Wert wird am Ausgang des Drucksensors 75 an eine Auswertungseinheit 77 übergeben, die ihrerseits mit einer Anzeigeeinheit 79 in Verbindung steht. Die erfassten Druckwerte werden ausgewertet und in aufbereiteter Form einem dem Katheter 60 handhabenden Operateur angezeigt, so dass dieser über Wandkontakte des Katheters im Körper des Patienten sowie die entsprechenden Druckkräfte informiert wird.

Fig. 6B zeigt eine andere Katheteranordnung mit einem Katheter 60', welches anstelle eines inflatierbaren Ballons als Deformationskörper einen Schaumstoffkörper 61' aufweist. Da hier keine Fluidversorgung für den Deformationskörper benötigt wird, umfasst der Außenschlauch 67k' einen Fluidkanal, und am proximalen Ende sind weder ein Fluidanschluss noch eine Fluidquelle vorgesehen.

Eine Erfassung der Deformation des Deformationskörpers 61'erfolgt über zwei Messelektrodenflächen 66a, 66b im distalen bzw. proximalen Bereich des Deformationskörpers, die über Messleitungen 68a bzw. 68b an eine Messstromversorgung 72 mit zugeordnetem Stromfühler 74 angeschlossen sind. Dem Stromfühler 74 sind, analog zur Ausführung nach Fig. 6A, eine Auswertungseinheit 76 sowie schließlich eine Anzeigeeinheit 79 zur Bereitstellung von Wandkontakt-Informationen für den Operateur nachgeschaltet. Eine Deformation des Deformationskörpers 61' führt zu einer Verringung des Abstandes zwischen den Messelektroden 66a, 66b und zugleich einer Komprimierung des Schaums, die sich ein einer Änderung des Widerstands im Strompfad zwischen den Messelektroden und somit in einer Änderung der Stromstärke ausdrückt. Deren Auswertung liefert die benötigte Information über das Bestehen eines Wandkontaktes und dessen Intensität.

Die Ausführung der Erfindung ist nicht auf die oben beschriebenen Beispiele und hervorgehobenen Aspekte beschränkt, sondern ebenso in einer Vielzahl von Abwandlungen möglich, die im Rahmen fachgemäßen Handelns liegen.

## Patentansprüche

1. Katheter mit einem langgestreckten Katheterkörper, der in Bezug auf eine Gebrauchslage ein distales und ein proximales Ende hat, wobei der Katheterkörper einen Innenschlauch und einen diesem gegenüber verschiebbaren Außenschlauch umfasst und am distalen Ende ein Deformationskörper angeordnet ist, der in seinem distalen Bereich mit dem distalen Ende des Innenschlauches und in seinem proximalen Bereich mit dem distalen Ende des Außenschlauches verbunden ist.

2. Katheter nach Anspruch 1, wobei der Deformationskörper als Ballon mit Fluidfüllung ausgebildet ist.

3. Katheter nach Anspruch 2, wobei der Ballon über einen Fluidkanal im Katheterkörper in Fluidverbindung mit mindestens einem Fluidanschluss am proximalen Ende des Katheters steht.

4. Katheter nach Anspruch 2 oder 3, wobei der Ballon mit einem elastisch kompressiblen Fluid gefüllt ist.

5. Katheter nach Anspruch 2 oder 3, wobei der Ballon mit einem inkompressiblen Fluid gefüllt ist.

6. Katheter nach Anspruch 1, wobei der Deformationskörper aus einem elastisch kompressiblen Material gebildet ist.

7. Katheter nach einem der vorangehenden Ansprüche, wobei der Deformationskörper eine im Wesentlichen axial wirkende Druckfedereinrichtung, insbesondere eine Metall- oder Kunststofffeder, aufweist.

8. Katheter nach einem der vorangehenden Ansprüche, wobei der Deformationskörper eine formelastische Hülle oder Mantelschicht aufweist.

9. Katheter nach einem der vorangehenden Ansprüche, wobei der Deformationskörper im entlasteten Zustand einen mindestens abschnittweise größeren Durchmesser als der Außenschlauch hat.

10. Katheter nach einem der vorangehenden Ansprüche, wobei der Deformationskörper, insbesondere elektrisch oder optisch wirkende, Messmittel oder einen Messanschluss zur Erfassung einer auf ihn ausgeübten Andruckkraft aufweist.

11. Katheter nach einem der vorangehenden Ansprüche, wobei der Deformationskörper mehrteilig aus mehreren Kammern oder Teilkörpern ausgebildet ist, wobei die Kammern oder Teilkörper unterschiedliches Deformationsverhalten und/oder separate Messmittel oder -anschlüsse zur Erfassung einer spezifisch auf sie ausgeübten Andruckkraft aufweisen.

12. Katheter nach Anspruch 11, wobei die Kammern jeweils über einen Fluidkanal im Katheterkörper in Fluidverbindung mit einem Fluidanschluss am proximalen Ende des Katheters stehen oder die Teilkörper optisch oder elektrisch wirkende Messmittel oder -anschlüsse aufweisen.

13. Katheter nach einem der vorangehenden Ansprüche, ausgebildet als Elektrodenleitung mit mindestens einer auf dem Deformationskörper oder am distalen Ende des Innenschlauches, umgeben vom Deformationskörper, angeordneten Elektrode.

14. Katheter nach Anspruch 13, wobei die oder mindestens eine Elektrode elastisch deformierbar, insbesondere aus leitfähigem Kunststoff gebildet, ist.

15. Katheteranordnung mit einem Katheter nach einem der Ansprüche 3 - 14 und einer mit dem oder jedem proximalen Fluidanschluss oder dem oder jedem sonstigen Messmittel oder -anschluss verbundenen Messeinrichtung zur Bestimmung einer auf den Deformationskörper oder Teilbereiche desselben einwirkenden Andruckkraft.
